# EUROPÄISCHE PATENTSCHRIFT

(11) **EP 1 853 292 B1**
(45) Veröffentlichungstag und Bekanntmachung des Hinweises auf die Patenterteilung: **10.12.2008**
(21) Anmeldenummer: 06706944.3
(22) Anmeldetag: 14.02.2006
(51) Int. Cl.: A61K 38/06

(54) **PHARMAZEUTISCHE ZUSAMMENSETZUNG ENTHALTEND GLUTATHION VERBINDUNGEN UND BETALAINE ZUR PRÄVENTION UND ZUR BEKÄMPFUNG VON KREBS**
PHARMACEUTICAL COMPOSITION COMPRISING GLUTATHIONE COMPOUNDS AND BETALAIN FOR THE PREVENTION AND TREATMENT OF CANCER
COMPOSITION PHARMACEUTIQUE CONTENANT DES COMPOSES DE GLUTATHIONE ET DE LA BETALAINE POUR LA PREVENTION ET LE TRAITEMENT DU CANCER

(30) Priorität: 14.02.2005 DE 202005002324 U
(43) Veröffentlichungstag der Anmeldung: 14.11.2007
(73) Patentinhaber: Gernot, Treusch, Falconera 59 17487 Empuria Brava (ES)
(72) Erfinder: Gernot, Treusch, Falconera 59 17487 Empuria Brava (ES)
(74) Vertreter: Lieck, Hans-Peter
(86) Internationale Anmeldenummer: PCT/EP2006/001338
(87) Internationale Veröffentlichungsnummer: WO 2006/084768

(56) Entgegenhaltungen:
- US-B1- 6 312 697
- DATABASE BIOSIS [Online] BIOSCIENCES INFORMATION SERVICE, PHILADELPHIA, PA, US; 1996, DONNERSTAG BARBARA ET AL: "Reduced glutathione and S-acetylglutathione as selective apoptosis-inducing agents in cancer therapy" XP002387578 Database accession no. PREV199799412286 & CANCER LETTERS, Bd. 110, Nr. 1-2, 1996, Seiten 63-70, ISSN: 0304-3835
- DATABASE BIOSIS [Online] BIOSCIENCES INFORMATION SERVICE, PHILADELPHIA, PA, US; Januar 2002 (2002-01), LOCIGNO ROBERTO ET AL: "S-acetyl-glutathione selectively induces apoptosis in human lymphoma cells through a GSH-independent mechanism" XP002387579 Database accession no. PREV200200099524 & INTERNATIONAL JOURNAL OF ONCOLOGY, Bd. 20, Nr. 1, Januar 2002 (2002-01), Seiten 69-75, ISSN: 1019-6439
- DATABASE BIOSIS [Online] BIOSCIENCES INFORMATION SERVICE, PHILADELPHIA, PA, US; November 2003 (2003-11), AZUINE MAGNUS ET AL: "Chemopreventive effects of betanin on ultraviolet-B-induced skin cancer in SENCAR mice." XP002387580 Database accession no. PREV200400077663 & CANCER EPIDEMIOLOGY BIOMARKERS & PREVENTION, Bd. 12, Nr. 11 Part 2, November 2003 (2003-11), Seite 1343s, SECOND ANNUAL AACR INTERNATIONAL CONFERENCE ON FRONTIERS IN CANCER PREVENTION RESEARCH : GENETICS, R; PHOENIX, ARIZONA, USA; OCTOBER 26-30, 2003 ISSN: 1055-9965
- DATABASE BIOSIS [Online] BIOSCIENCES INFORMATION SERVICE, PHILADELPHIA, PA, US; Februar 2003 (2003-02), KAPADIA GOVIND J ET AL: "Chemoprevention of DMBA-induced UV-B promoted, NOR-1-induced TPA promoted skin carcinogenesis, and DEN-induced phenobarbital promoted liver tumors in mice by extract of beetroot." XP002387581 Database accession no. PREV200300149552 & PHARMACOLOGICAL RESEARCH, Bd. 47, Nr. 2, Februar 2003 (2003-02), Seiten 141-148, ISSN: 1043-6618

## Beschreibung

Es ist bekannt, reduziertes Glutathion oder bestimmte Thiol-Derivate des Glutathions in Kombination mit Anthocyanen oder Anthocyanidinen u.a. zur Krebsbekämpfung einzusetzen (EP 0 545 972 B1). In dieser Kombination wird die pharmazeutische Wirkung der Glutathion-Verbindung durch das Anthocyan oder Anthocyanidin synergistisch verstärkt.

Es wurde nun erkannt, daß überraschenderweise die Kombination von reduziertem Glutathion oder bestimmten Thiol-Verbindungen des Glutathions mit Betalainen oder deren Aglykonen eine deutlich ausgeprägtere Wirkung gegen Krebs zu haben verspricht als die vorbekannte Glutathion/Anthocyan-Kombination. Die Wirkung ist besonders ausgeprägt, wenn entgegen der durch den Stand der Technik nahegelegten Vermutung, auf die zusätzliche Verwendung oder Anwesenheit von Anthocyanen und Anthocyanidinen in der Kombination gänzlich verzichtet wird.

Dementsprechend wird erfindungsgemäß eine pharmazeutische Zusammensetzung zur Krebsbekämpfung vorgeschlagen, die als pharmazeutisch wirksame Substanzen reduziertes Glutathion und/oder S-Methylglutathion und/oder S-Ethylglutathion und/- oder S-Acetylglutathion und/oder S-Phosphorsäure-Glutathion sowie mindestens eine Verbindung aus der Gruppe der Betalaine und deren Aglykone enthält, wobei vorzugsweise gegebenenfalls vorhandene weitere Wirkstoffe nicht zur Gruppe der Anthocyane und deren Aglykone gehören.

Gegenstand der Erfindung ist ferner die Verwendung von reduziertem Glutathion und/oder S-Methylglutathion und/oder S-Ethylglutathion und/oder S-Acetylglutathion und/oder S-Phosphorsäureglutathion in Kombination mit mindestens einer Verbindung aus der Gruppe der Betalaine und deren Aglykone, vorzugsweise ohne Mitverwendung von Anthocyanen oder deren Aglykonen, zur therapeutischen einschließlich der dietätisch vorbeugenden Behandlung des menschlichen oder tierischen Körpers gegen Krebs.

Glutathion (Gamma-Glutamyl-Cysteinyl-Glycin) ist ein Tripeptid aus den drei Aminosäuren Glutaminsäure, Cystein und Glyzin, das in seiner reduzierten Form (G-SH) in den meisten Zellen von Menschen und Säugetieren vorkommt. Seine Strukturformel ist in der EP 0 545 972 B1 wiedergegeben.

Das reduzierte Glutathion kann in der erfindungsgemäßen Zusammensetzung ganz oder teilweise durch bestimmte Thiol-Derivate des Glutathions ersetzt sein, nämlich durch den MonoMethyl-Thio-Ester, den Mono-Ethyl-Thio-Ester, den Mono-Acetyl-Thio-Ester und/oder den Mono-Phosphorsäure-Thio-Ester des Glutathions. Die Strukturformeln dieser Derivate sind ebenfalls in der EP 0 545 972 B1 angegeben.

Die Thiol-Derivate des Glutathions haben gegenüber dem reduzierten Glutathion in manchen Anwendungsfällen den Vorteil höherer Bioverfügbarkeit, weil ihre Fähigkeit zur Permeation durch biologische Membranen hoch ist und bei ihnen die für den therapeutischen Effekt wichtige SH-Gruppe des Glutathions auf dem Weg durch die biologischen Kömpartimente bis zum gewünschten Wirkort geschützt ist.

Alternativ zu oder neben den Thiol-Derivaten des Glutathions kommen auch bestimmte Glutathion-Ester in Frage, bei denen die Veresterung nicht an der Thiolgruppe, sondern an der Carboxylgruppe des Glycylrestes stattfindet. Es handelt sich um Mono-Methylglutathion-Ester, Mono-Ethylglutathion-Ester, Mono-Acetylglutathion-Ester, Mono-Phosphorsäureglutathion-Ester und/oder Isopropylglutathion-Ester. Auch diese Derivate sind lipophiler als G-SH und können daher in die Zellmembranen besser permeieren.

Betalaine sind Blüten- und Frucht-Farbstoffe, die sich von der Batalaminsäure ableiten. Zu ihnen gehören die rotvioletten Betacyane aus roten Rüben (Beta Vulgaris) und die gelben Betaxanthine aus Kakteen. Typische Vertreter der Betacyane sind Betanin, Isobetanin, Präbetanin und Isopräbetanin. Das Aglykon des Betanins ist das Betanidin. Typische Vertreter der Betaxanthine sind Vulgarxanthin I und Vulgarxanthin II. Die Betalaine werden in erster Linie durch Isolierung aus den entsprechenden Blüten und Früchten gewonnen; durch verschiedene Tests lassen sie sich von den Anthocyanen deutlich unterscheiden, vgl. hierzu Schweppe, Handbuch der Naturfarbstoffe. Vorkommen, Verwendung, Nachweis. Landsberg: ecomed 1972, Seite 407 bis 409.

Erfindungsgemäß wird eine Zusammensetzung bevorzugt, deren Wirkstoffe reduziertes Glutathion und/oder S-Acetylglutathion sowie Betanin und/oder Betanidin sind. Ein Verfahren zur Herstellung von S-Acetylglutathion ist in der US 2,760,956 offenbart. Betanin, auch Beetenrot genannt (C₂₄H₂₆N₂O₁₃), ist als Lebensmittel- und Kosmetik-Farbstoff zugelassen und wird aus der Roten Rübe bzw. Roten Beete gewonnen.

Die erfindungsgemäße Zusammensetzung kann als zusätzliche Wirkstoffe FAD (Flavin-Adenin-Dinucleotid) und/oder Genistein, z. B. gewonnen aus Sojabohnen, enthalten.

Als weiterer, zusätzlicher Wirkstoff in der erfindungsgemäßen Zusammensetzung kommt insbesondere Superoxiddismutase (SOD) in Frage, vorzugsweise in einer Tagesdosis von 500 mg bis 1500 mg.

Die erfindungsgemäße Zusammensetzung kann bei allen Krebsarten und Metastasen zum Einsatz kommen, in der oralen, nasalen, baccalen oder sublingualen Applikation, als Injektion, als Infusion oder als Suppositorium. Durch Einschluß der pharmazeutisch wirksamen Substanzen in Liposome und/oder Cyclodextrine kann die erfindungsgemäße Zusammensetzung galenisch noch weiter verbessert werden.

Mittels solcher Träger kann in an sich bekannter Weise die Toxizität der Wirkstoffe herabgesetzt und ihre Wirksamkeit durch gesteuerte Freisetzung genau am Ort des Tumors oder der Metastase erhöht werden.

Durchgeführt wurden in vitro-Untersuchungen zur Wirkung von reduziertem Glutathion (G-SH) sowie Anthocyanen und Betacyanen, jeweils einzeln und in verschiedenen Kombinationen miteinander, auf Tumorzellinien, nämlich H69: humanes kleinzelliges Lungenkarzinom. Verwendet wurden G-SH und Betalaine als Trockenextrakt der Beta Vulgaris (Produkt Betatom der Firma Sprüh-Atom Schumann & Sohn GmbH, Karlsruhe), sowie - zum Vergleich - Anthocyane als Trockenextrakte von Cassis (schwarze Johannisbeere) und Vaccinium (Heidelbeere). Die Tumorzelllinien wurden in vitro in DMEM mit 10% FCS ausgesät. Die Endkonzentration der zugegebenen Substanzen G-SH, Betatom, Cassis und Vaccinium war 1 mM. Die Apoptose wurde mit einem Annexin-Testsystem gemessen. Alle Versuche wurden einmal wiederholt. Die Wirkung auf H69 Tumorzellen ist in der nachfolgenden Tabelle dargestellt. Nach einer zehntägigen Behandlung der Zellen war sowohl die Zahl der apoptotischen als auch der defekten Zellen gegenüber unbehandelten Kontrollen deutlich erhöht. Die Kombination von GSH und Betatom ohne Anwesenheit von Anthocyanen (Cassis und Vaccinium) hatte dabei die signifikant größte Wirkung.

Ein typisches Präparat zur Krebsbekämpfung nach der Erfindung liegt in Form von Tabletten zur oralen Einnahme vor, die jeweils 200 mg Glutathion und 200 mg Betatom als alleinige Wirkstoffe enthalten und in einer Dosierung von 5 - 10 Tabletten pro Tag gegeben werden.

### Apoptotische und defekte H69 Tumorzellen nach 10 Tagen Behandlung

| | **GSH** | **Cassis** | **Vaccinium** | **Betatom** | **Vaccinium +GSH** | **Cassis +GSH** | **Cassis +Vaccinium +GSH** | **Betatom +Cassis +GSH** | **Betatom +Vaccinium +GSH** | **Betatom +GSH** | **Kontrolle** |
|---|---|---|---|---|---|---|---|---|---|---|---|
| apoptotische Zellen (%Gesamtzellen) | 21,9 | 26,5 | 28,7 | 48,9 | 34,6 | 35,4 | 63,0 | 65,6 | 66,7 | 86,6 | 27,3 |
| Veränderung gegenüber Kontrolle (%) | -5 | -0,7 | 1,4 | 21,7 | 8,2 | 7,4 | 35,8 | 38,4 | 39,5 | 59,3 | |
| | | | | | | | | | | | |
| defekte Zellen (%Gesamtzellen) | 37,9 | 45,2 | 45,4 | 58,0 | 51,3 | 54,3 | 61,0 | 68,7 | 72,5 | 88,9 | |
| Veränderung gegenüber Kontrolle (%) | 8,9 | 16,2 | 16,3 | 28,9 | 22,2 | 25,2 | 32,0 | 39,6 | 43,5 | 59,8 | |

## Patentansprüche

1. Pharmazeutische Zusammensetzung zur Prävention und zur Bekämpfung von Krebs,
enthaltend als pharmazeutisch wirksame Substanzen reduziertes Glutathion und/oder S-Methylglutathion und/oder S-Ethylglutathion und/oder S-Acetylglutathion und/oder S-Phosphorsäureglutathion und/oder
Mono-Methylglutathion-Ester und/oder Mono-Ethylglutathion-Ester und/oder Mono-Acetylglutathion-Ester und/oder Mono-Phosphorsäureglutathion-Ester und/oder Isopropylglutathion-Ester
sowie mindestens eine Verbindung aus der Gruppe der Betalaine und deren Aglykone.

2. Pharmazeutische Zusammensetzung nach Anspruch 1,
bei welcher gegebenenfalls vorhandene weitere Wirkstoffe nicht zur Gruppe der Anthocyane und deren Aglykone gehören.

3. Pharmazeutische Zusammensetzung zur Prävention und zur Bekämpfung von Krebs,
enthaltend als pharmazeutisch wirksame Substanzen ausschließlich reduziertes Glutathion und/oder S-Methylglutathion und/- oder S-Ethylglutathion und/oder S-Acetylglutathion und/oder S-Phosphorsäureglutathion sowie mindestens eine Verbindung aus der Gruppe der Betalaine und deren Aglykone.

4. Zusammensetzung nach Anspruch 2 oder 3,
enthaltend als pharmazeutisch wirksame Substanzen reduziertes Glutathion und/oder S-Methylglutathion und/oder S-Ethylglutathion und/oder S-Acetylglutathion und/oder S-Phosphorsäureglutathion sowie mindestens eine Verbindung aus der Gruppe der Betacyane und deren Aglykone.

5. Zusammensetzung nach Anspruch 4,
enthaltend reduziertes Glutathion und/oder S-Acetylglutathion sowie Betanin und/oder Betanidin.

6. Zusammensetzung nach Anspruch 1, 2, 4 oder 5,
enthaltend Superoxiddismutase als weitere pharmazeutisch wirksame Substanz.

7. Zusammensetzung nach einem der Ansprüche 1 bis 6,
bei welcher die pharmazeutisch wirksamen Substanzen ganz oder teilweise in Liposomen und/oder Cyclodextrinen als molekulare Träger eingeschlossen sind.

8. Verwendung einer pharmazeutischen Zusammensetzung nach einem der Ansprüche 1 bis 7 zur Herstellung eines Medikamentes für die therapeutische Behandlung des menschlichen oder tierischen Körpers gegen Krebs.

9. Verwendung nach Anspruch 8
ohne Mitverwendung von Anthocyanen und deren Aglykonen.

## Claims

1. A pharmaceutical composition for the prevention and control of cancer,
containing, as pharmaceutically active substances, reduced glutathione and/or S-methyl glutathione and/or S-ethyl glutathione and/or S-acetyl glutathione and/or S-phosphoric glutathione and/or
monomethyl glutathione ester and/or monoethyl glutathione ester and/or monoacetyl glutathione ester and/or monophosphoric glutathione ester and/or isopropyl glutathione ester,
and at least one compound of the group consisting of the betalaines and the aglycones thereof.

2. The pharmaceutical composition as claimed in claim 1, wherein any other active substances it may include do not belong to the group of anthocyan and the aglycones thereof.

3. A pharmaceutical composition for the prevention and control of cancer,
containing, as pharmaceutically active substances, exclusively reduced glutathione and/or S-methyl glutathione and/or S-ethyl glutathione and/or S-acetyl glutathione and/or S-phosphoric glutathione, as well as at least one compound of the group consisting of the betalains and the aglycones thereof.

4. The composition as claimed in claim 2 or 3, containing, as pharmaceutically active substances, reduced glutathione and/or S-methyl glutathione and/or S-ethyl glutathione and/or S-acetyl glutathione and/or S-phosphoric glutathione, as well as at least one compound of the group consisting of betacyans and the aglycones thereof.

5. The composition as claimed in claim 4, containing reduced glutathione and/or S-acetyl glutathione as well as betanin and/or betanidin.

6. The composition as claimed in claim 1, 2, 4, or 5, containing superoxide dismutase as a further pharmaceutically active substance.

7. The composition as claimed in any one of claims 1 to 6, wherein the pharmaceutically active substance is included completely or partly in liposomes and/or cyclodextrins as molecular substrates.

8. Use of a pharmaceutical composition as claimed in any one of claims 1 to 7 or the pharmaceutically active substances thereof in combination with one another for treating a human or animal body against cancer.

9. The use as claimed in claim 8 without including the use of anthocyans and the aglycones thereof.

## Revendications

1. Composition pharmaceutique destinée à la prévention et au traitement du cancer,
contenant en tant que substances pharmaceutiquement actives du glutathion à l'état réduit et/ou du S-méthylglutathion et/ou du S-éthylglutathion et/ou du S-acétylglutathion et/ou du glutathion à l'état réduit S-acide phosphorique et/ou
de l'ester de mono-méthylglutathion et/ou de l'ester de mono-éthylglutathion et/ou de l'ester de mono-acétylglutathion et/ou de l'ester de glutathion à l'état réduit acide phosphorique et/ou de l'ester de l'isopropylglutathion
ainsi qu'au moins un composé issu du groupe des bétalaïnes et de leurs aglycones.

2. Composition pharmaceutique selon la revendication 1,
pouvant contenir d'autres principes actifs n'appartenant pas au groupe des anthocyanes et de leurs aglycones.

3. Composition pharmaceutique destinée à la prévention et au traitement du cancer,
ne contenant, en tant que substances pharmaceutiquement actives, que du glutathion à l'état réduit et/ou du S-méthylglutathion et/ou du S-éthylglutathion et/ou du S-acétylglutathion et/ou du glutathion à l'état réduit S-acide phosphorique ainsi qu'au moins un composé issu du groupe des bétalaïnes et de leurs aglycones.

4. Composition selon les revendications 2 ou 3,
contenant en tant que substances pharmaceutiquement actives que du glutathion à l'état réduit et/ou du S-méthylglutathion et/ou du S-éthylglutathion et/ou du S-acétylglutathion et/ou du glutathion S-acide phosphorique ainsi qu'au moins un composé issu du groupe des bétacyanes et de leurs aglycones.

5. Composition selon la revendication 4,
contenant du glutathion à l'état réduit et et/ou du S-acétylglutathion ainsi que de la bétanine et/ou du bétanidine.

6. Composition selon les revendications 1, 2, 4 ou 5,
contenant, en tant que substance pharmaceutiquement active supplémentaire, de la superoxyde dismutase.

7. Composition selon l'une des revendications 1 à 6,
au sein de laquelle les substances pharmaceutiquement actives sont en partie ou en totalité encapsulées dans des liposomes et/ou des cyclodextrines servant de supports moléculaires.

8. Utilisation d'une composition pharmaceutique selon l'une des revendications 1 à 7 pour produire un médicament destiné au traitement thérapeutique anticancéreux du corps humain ou animal.

9. Utilisation selon la revendication 8
sans mise en oeuvre d'anthocyanes et de leurs aglycones.
